# EUROPEAN PATENT APPLICATION

(11) **EP 2 169 429 A2**
(43) Date of publication of application: **31.03.2010**
(21) Application number: 09011446.3
(22) Date of filing: 07.09.2009
(51) Int. Cl.: G01T 1/20, G01T 1/29

(54) **Radiation detecting apparatus and radiation image capturing system**

(30) Priority: 30.09.2008 JP 2008254869
(71) Applicant: Fujifilm Corporation, Tokyo 106-8620 (JP)
(72) Inventor: Nishino, Naoyuki, Ashigarakami-gun, Kanagawa 258-8538 (JP); Ohta, Yasunori, Ashigarakami-gun, Kanagawa 258-8538 (JP); Seto, Yasuhiro, Ashigarakami-gun, Kanagawa 258-8538 (JP); Tsubota, Keiji, Ashigarakami-gun, Kanagawa 258-8538 (JP); Yoshida, Yutaka, Ashigarakami-gun, Kanagawa 258-8538 (JP); Kito, Eiichi, Ashigarakami-gun, Kanagawa 258-8538 (JP); Imai, Shinji, Ashigarakami-gun, Kanagawa 258-8538 (JP)
(74) Representative: Klunker . Schmitt-Nilson . Hirsch

(57) **Abstract**

A radiation detecting apparatus (18) includes a flexible radiation detector (30) for detecting radiation (12) that has passed through a subject (14) and converting the detected radiation (12) into radiation image information. The flexible radiation detector (30) includes a flexible substrate (40), and a plurality of solid block bodies (32) mounted in a two-dimensional array on the flexible radiation detector (30). The radiation detecting apparatus is capable of avoiding excessive deformations in the radiation detector, and prevents image distortions of the radiation image information. The radiation detecting apparatus also prevents disconnections in the radiation detector. There is also provided a radiation image capturing system (10) incorporating such a radiation detecting apparatus (18).

## Description

### BACKGROUND OF THE INVENTION

### Field of the Invention:

The present invention relates to a radiation detecting apparatus incorporating therein a radiation detector for detecting radiation that has passed through a subject and converting the detected radiation into radiation image information, and a radiation image capturing system incorporating such a radiation detecting apparatus. Description of the Related Art:

In the medical field, there have widely been used radiation image capturing systems that apply a radiation to a subject and guide radiation that has passed through the subject to a radiation conversion panel, which captures a radiation image from the radiation. Known forms of radiation conversion panels include a conventional radiation film for recording a radiation image by way of exposure, and a stimulable phosphor panel for storing radiation energy representing a radiation image in a phosphor and reproducing the radiation image as stimulated light by applying stimulating light to the phosphor. The radiation film with the radiation image therein is supplied to a developing device in order to develop the radiation image, or the stimulable phosphor panel is supplied to a reading device in order to read the radiation image as a visible image.

In an operating room or the like, it is necessary to read recorded radiation image information from a radiation conversion panel immediately after the radiation image information has been captured therein for the purpose of quickly and appropriately treating the patient. As a radiation detector which meets such a requirement, there has been developed a radiation detector for directly converting radiation into electric signals, or for temporarily converting radiation into visible light with a scintillator and converting the visible light into electric signals to read detected radiation image information with solid-state detectors.

Heretofore, there has been proposed an X-ray diagnostic apparatus having a flexible X-ray solid-state detector, which is capable of matching itself to any desired surface shape (see, for example, Japanese Laid-Open Patent Publication No. 2003-070776).

There has also been proposed an apparatus including a phosphorescent layer or a scintillator layer disposed on a support body, which is made flexible by means of pits or grooves defined therein (see, for example, Japanese Laid-Open Patent Publication No. 2005-049341).

Since the X-ray solid-state detectors disclosed in Japanese Laid-Open Patent Publication No. 2003-070776 and Japanese Laid-Open Patent Publication No. 2005-049341 are flexible, they can match themselves to various surface shapes for detecting X-rays that have passed through the surface shapes. However, each of the disclosed X-ray solid-state detectors is disadvantageous since it is flexibly deformed to local convexities and concavities, and therefore it is hard to correct radiation image information that has been generated from the deformed X-ray solid-state detector. Specifically, since the X-ray solid-state detector develops local convexities and concavities due to surface irregularities of the subject to be imaged against which the X-ray solid-state detector is held, the X-ray solid-state detector becomes physically distorted into complex shapes, which cause the radiation image information generated thereby to develop corresponding image distortions, which may not be corrected properly.

In addition, inasmuch as the disclosed X-ray solid-state detectors are susceptible to excessive deformation, they may become plastically deformed during use. In particular, the X-ray solid-state detector disclosed in Japanese Laid-Open Patent Publication No. 2005-049341 may become split at the grooves formed therein, tending to cause disconnections.

### SUMMARY OF THE INVENTION

It is an object of the present invention to provide a radiation detecting apparatus which can avoid excessive deformation of a radiation detector, and which does not cause image distortions in the radiation image information generated by the radiation detector. The radiation detecting apparatus also prevents disconnections from occurring in the radiation detector, has increased reliability during operation, and can be used and stored highly conveniently.

Another object of the present invention is to provide a radiation image capturing system incorporating such a radiation detecting apparatus, which does not need to take into account image distortions caused by deformations of the radiation detector during the image processing operation, which provides an inspection space in which the radiation detecting apparatus can be compactly stored, and which allows an operator to work smoothly when capturing radiation images.

A radiation detecting apparatus according to a first aspect of the present invention includes a flexible radiation detector for detecting radiation that has passed through a subject and converting the detected radiation into radiation image information. The flexible radiation detector includes a flexible substrate, and a plurality of solid block bodies mounted in a two-dimensional array on the flexible radiation detector.

A radiation image capturing system according to a second aspect of the present invention includes a radiation detecting apparatus according to the first aspect of the present invention, a radiation source for outputting radiation, and a control apparatus for controlling the radiation source and the radiation detecting apparatus.

According to the present invention, the radiation detecting apparatus can avoid excessive deformations of the radiation detector, and does not cause image distortions in the radiation image information. The radiation detecting apparatus also prevents disconnections in the radiation detector, has increased reliability during operation, and can be used and stored highly conveniently.

According to the present invention, the radiation image capturing system, which incorporates the above radiation detecting apparatus therein, does not need to take into account image distortions that otherwise could be caused by deformations of the radiation detector during image processing, which provides an inspection space in which the radiation detecting apparatus can be compactly stored, and which allows an operator to work smoothly during capturing of radiation images.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a block diagram of a radiation detecting apparatus and a radiation image capturing system according to an embodiment of the present invention;
FIG. 2 is a perspective view of a first radiation detecting apparatus in the radiation image capturing system shown in FIG. 1;
FIG. 3 is a fragmentary vertical cross-sectional view of the first radiation detecting apparatus;
FIG. 4 is a block diagram of a circuit arrangement of a radiation detector in the first radiation detecting apparatus;
FIG. 5 is a diagram showing a relationship between first solid block bodies and an array of pixels of the radiation detector;
FIG. 6 is a diagram showing an example of interconnections to the radiation detector and an installed state of electronic circuits;
FIG. 7 is a fragmentary cross-sectional view, partially omitted from illustration, showing an electronic circuit installed in the radiation detector;
FIG. 8 is a fragmentary cross-sectional view, partially omitted from illustration, showing an electronic circuit housed in a first solid block body according to another example;
FIG. 9 is a diagram showing an example of grouped interconnections between gate lines and divided row selection scanners corresponding to first solid block bodies;
FIG. 10 is a perspective view of a first solid block body having separate grids disposed therein;
FIG. 11 is a diagram showing the first radiation detecting apparatus in a spirally wound state;
FIG. 12 is a fragmentary cross-sectional view showing another example of a portion of the first radiation detecting apparatus; and
FIG. 13 is a perspective view of a second radiation detecting apparatus.

### DESCRIPTION OF THE PREFERRED EMBODIMENTS

Like or corresponding parts are denoted by like or corresponding reference characters throughout the views.

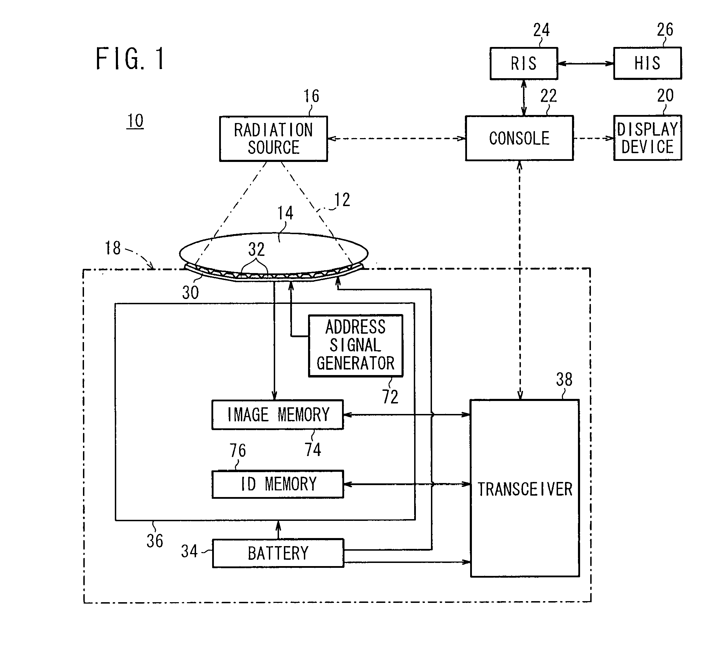

Radiation detecting apparatus according to embodiments of the present invention, and a radiation image capturing system incorporating such radiation detecting apparatus therein, shall be described below with reference to FIGS. 1 through 13.

FIG. 1 shows in block form a radiation image capturing system 10, comprising a radiation source 16 for applying radiation 12 at a dosage according to image capturing conditions to a subject 14 such as a patient, a radiation detecting apparatus 18 according to the present invention, a display device 20 for displaying radiation image information based on the radiation 12 detected by the radiation detecting apparatus 18, and a console (control apparatus) 22 for controlling the radiation detecting apparatus 18, the radiation source 16, and the display device 20. Signals are sent and received between the console 22, the radiation detecting apparatus 18, the radiation source 16, and the display device 20 based on wireless LAN communications, according to UWB (Ultra-WideBand) technology or IEEE 802.11.a/g/n. The console 22 is connected to a radiology information system (RIS) 24, which generally manages radiation image information handled by the radiological department of the hospital, together with other information. The RIS 24 is connected to a hospital information system (HIS) 26, which generally manages medical information in the hospital.

As shown in FIG. 1, the radiation detecting apparatus 18 comprises a flexible radiation detector 30 for detecting radiation 12 that has passed through the subject 14 and converting the detected radiation 12 into radiation image information, and a plurality of solid block bodies 32 disposed in a two-dimensional matrix on the radiation detector 30.

The radiation detecting apparatus 18 also includes a battery 34 that serves as a power supply for the radiation detector 30, a controller 36 for energizing the radiation detector 30 with electric power supplied from the battery 34, and a transceiver 38 for sending and receiving signals, including information of the radiation 12 detected by the radiation detector 30, to and from the console 22. The battery 34 supplies electric power to the radiation detector 30, the controller 36, and the transceiver 38.

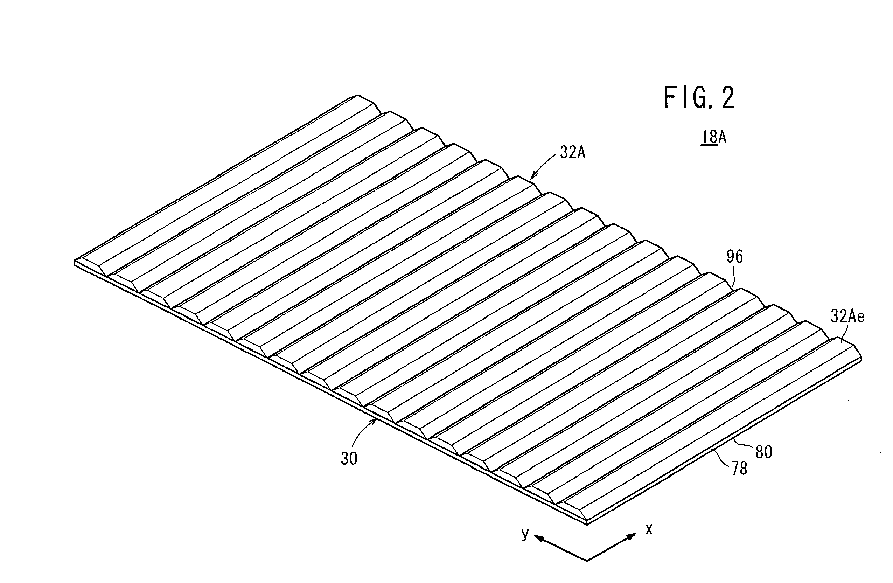

Specific examples of the radiation detecting apparatus 18 will be described below with reference to FIGS. 2 through 13.

As shown in FIG. 2, a radiation detecting apparatus according to a first specific example (hereinafter referred to as a first radiation detecting apparatus 18A) comprises the radiation detector 30 and a plurality of first solid block bodies 32A arrayed on the radiation detector 30.

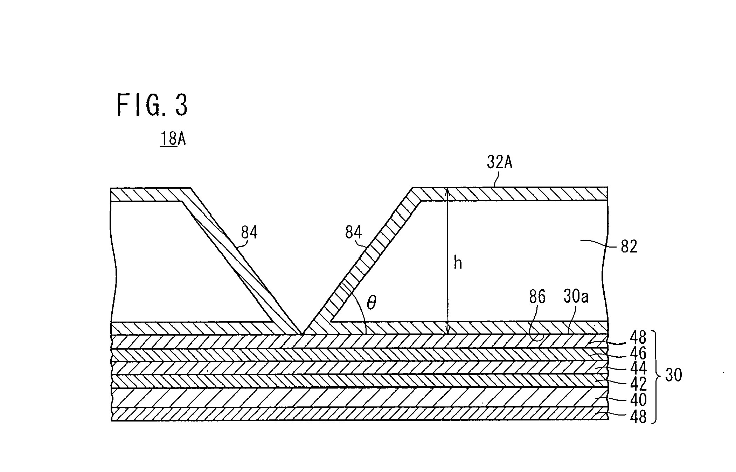

As shown in FIG. 3, the radiation detector 30 comprises a flexible substrate 40, a scintillator 42 disposed on the flexible substrate 40, a TFT layer 44 disposed on the scintillator 42, the TFT layer 44 comprising an array of thin film transistors (TFTs), and a photoelectric transducer layer 46 disposed on the TFT layer 44.

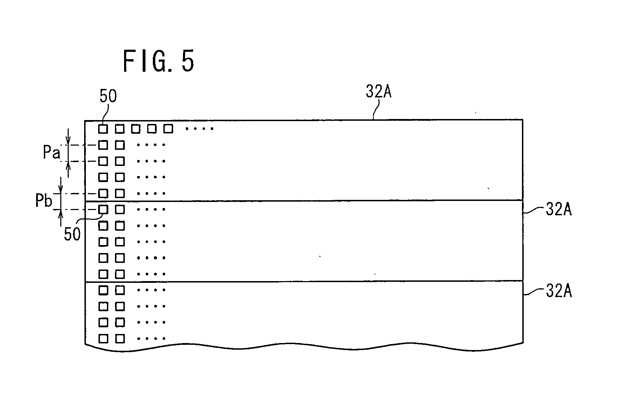

The scintillator 42 is made up of a phosphor, such as GOS (Gd₂O₂S) or CsI, for converting the radiation 12 (see FIG. 1) that has passed through the subject 14 into visible light. The TFT layer 44, which comprises an array of TFTs 54 (see FIG. 5), is permeable to radiation 12 and to visible light. The photoelectric transducer layer 46 includes solid-state detectors (hereinafter referred to as pixels) 50, made of a material such as amorphous silicon (a-Si) or the like, for converting the visible light into electric signals.

The flexible substrate 40 may be made of polyethylene terephthalate (PET), polyethylene naphthalate (PEN), polyimide (e.g., Kapton (registered trademark) of DuPont), polysulfone ether (PES), polycarbonate, or the like, as disclosed in Japanese Laid-Open Patent Publication No. 2003-070776.

The entire surface of the radiation detector 30 is covered with a light shield film 48, which prevents ambient light from entering the radiation detector 30. The light shield film 48 may be formed on the radiation detector 30 by a laminating process, a molding process, or the like.

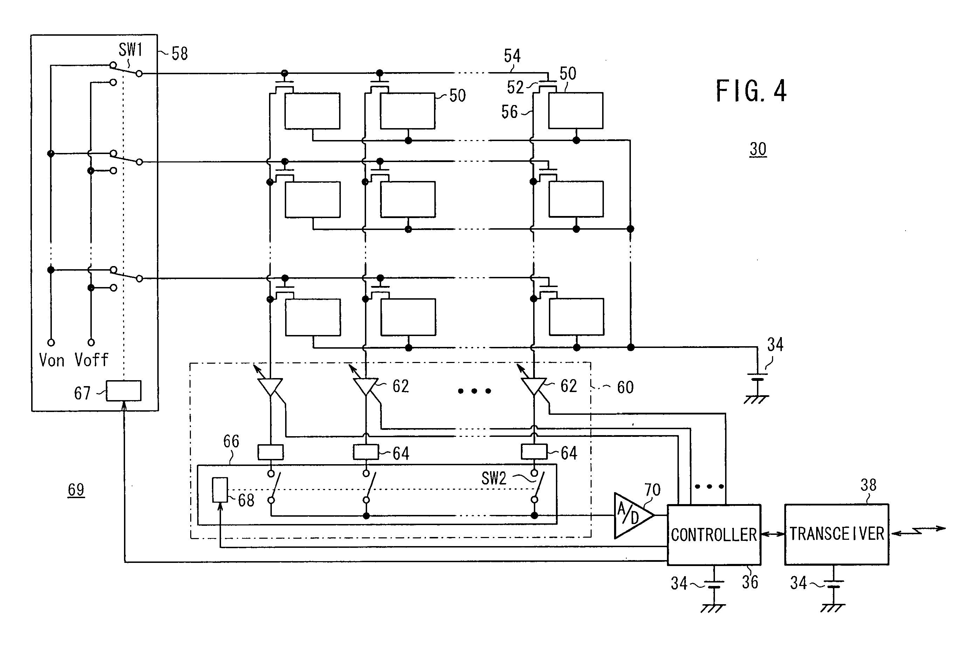

FIG. 4 shows in block form a circuit arrangement of the radiation detector 30. As shown in FIG. 4, the radiation detector 30 comprises an array of thin-film transistors (TFTs) 52 arranged in rows and columns (the TFT layer 44), with the photoelectric transducer layer 46 disposed on the array of TFTs 52. When radiation 12 is applied to the radiation detector 30, the radiation 12 is converted into visible light, which is applied to the photoelectric transducer layer 46. The photoelectric transducer layer 46 converts the visible light into electric signals, i.e., electric charges. The pixels 50 store the generated electric charges. Then, the TFTs 52 are turned on, each row at a time, in order to read the electric charges from the pixels 50 as an image signal.

The TFTs 52 connected to the respective pixels 50 are connected to respective gate lines 54 extending parallel to the rows, and to respective signal lines 56 extending parallel to the columns. The gate lines 54 are connected to a row selection scanner 58, and the signal lines 56 are connected to a column selection scanner 60. The column selection scanner 60 comprises a plurality of amplifiers 62 connected respectively to the signal lines 56, a plurality of sample and hold circuits 64 connected respectively to the amplifiers 62, and a multiplexer 66 for selectively scanning the signal lines 56.

The gate lines 54 are supplied with control signals Von, Voff from the row selection scanner 58 for turning on and off the TFTs 52 along the rows. The row selection scanner 58 comprises a plurality of switches SW1 for switching between the gate lines 54, and an address decoder 67 for outputting a selection signal for selecting one of the switches SW1 at a time. The address decoder 67 is supplied with an address signal from the controller 36.

The signal lines 56 are supplied with electric charges stored in the pixels 50 through the TFTs 52 arranged in the columns. The electric charges supplied to the signal lines 56 are amplified by the amplifiers 62 and supplied through the sample and hold circuits 64 to the multiplexer 66. The multiplexer 66 comprises a plurality of switches SW2 for successively switching between the signal lines 56, and an address decoder 68 for outputting a selection signal for selecting one of the switches SW2 at a time. The address decoder 68 is supplied with an address signal from the controller 36. The multiplexer 66 has an output terminal connected to an A/D converter 70. A radiation image signal generated by the multiplexer 66 based on the electric charges from the sample and hold circuits 64 is converted by the A/D converter 70 into a digital image signal representing radiation image information, which is supplied to the controller 36.

As shown in FIG. 1, the controller 36 of the first radiation detecting apparatus 18A comprises an address signal generator 72, an image memory 74, and an ID memory 76.

The address signal generator 72 supplies address signals to the address decoder 67 of the row selection scanner 58, and to the address decoder 68 of the multiplexer 66 of the radiation detector 30. The image memory 74 stores the radiation image information detected by the radiation detector 30. The ID memory 76 stores ID information for identifying the first radiation detecting apparatus 18A.

The transceiver 38 transmits the ID information stored in the cassette ID memory 76 and the radiation image information stored in the image memory 74 to the console 22 by way of wireless communications.

As shown in FIG. 2, each of the first solid block bodies 32A of the first radiation detecting apparatus 18A has an elongate rectangular shape as viewed in plan, which has a longer side 78 with a length is equal to the length of a side 80 of the radiation detector 30. The first solid block bodies 32A are arrayed in the direction indicated by the arrow y, which is perpendicular to the side 80 of the radiation detector 30. The first solid block bodies 32A are arrayed on a surface of the radiation detector 30 that is irradiated with radiation 12, i.e., an irradiated surface 30a, as shown in FIG. 3. As shown in FIG. 3, a heat insulator 82 of expanded polystyrene, air, or the like, is disposed in each of the first solid block bodies 32A.

Each of the first solid block bodies 32A has a tapered surface 84, which faces a corresponding tapered surface 84 of another first solid block body 32A disposed adjacent thereto. Such mutually facing tapered surfaces 84 are spaced progressively wider from each other in a direction away from the radiation detector 30.

The radiation detector 30 has pixels 50 arrayed in an area corresponding to each of the bottom surfaces 86 of the first solid block bodies 32A. As shown in FIG. 5, the pixels 50 are arrayed in an area corresponding to each of the bottom surfaces 86 of the first solid block bodies 32A at a pitch Pa. The closest ones of the pixels 50 in adjacent areas are arrayed at a pitch Pb, which is essentially the same as the pitch Pa.

When an external force is applied to the first radiation detecting apparatus 18A, the radiation detecting apparatus 18A becomes bent at boundaries between adjacent ones of the first solid block bodies 32A. More particularly, when the first radiation detecting apparatus 18A is bent in a direction that brings adjacent ones of the first solid block bodies 32A toward each other, the first radiation detecting apparatus 18A stops being bent, at a time when mutually facing tapered surfaces 84 of the adjacent first solid block bodies 32A come into contact with each other. If the first solid block bodies 32A have smaller widths, then the overall curvature of the first radiation detecting apparatus 18A is variable over a greater range. If the first solid block bodies 32A have greater widths, then the overall curvature of the first radiation detecting apparatus 18A is variable over a smaller range. The overall curvature of the first radiation detecting apparatus 18A may be varied within a desired range by changing the angle θ at which the tapered surfaces 84 are inclined, and by changing the height h of the first solid block bodies 32A.

When the first radiation detecting apparatus 18A is bent, the first radiation detecting apparatus 18A is deformed as a whole only in the direction indicated by the arrow y in FIG. 2, which is perpendicular to the longitudinal direction of the first solid block bodies 32A. The first radiation detecting apparatus 18A is not deformed in the direction indicated by the arrow x in FIG. 2, which is parallel to the longitudinal direction of the first solid block bodies 32A. Therefore, the first radiation detecting apparatus 18A can be placed in an orientation that more accurately matches the surface shape of the subject 14, which is of a columnar shape. For example, the first radiation detecting apparatus 18A may be set so as to cover an arm, a leg, or the torso of a subject 14, for thereby obtaining a large amount of radiation image information concerning the subject 14.

The first radiation detecting apparatus 18A is prevented from becoming further deformed after the adjacent tapered surfaces 84 of the first solid block bodies 32A come into contact with each other. Accordingly, the first radiation detecting apparatus 18A is prevented from becoming excessively deformed, thereby preventing the gate lines 54 and the signal lines 56 from becoming disconnected.

The pixels 50 that make up the photoelectric transducer layer 46 are positioned on bottom surfaces of the first solid block bodies 32A. When an external force is applied at a point on one of the first solid block bodies 32A, such an external force does not act as a concentrated load, but rather is distributed by the first solid block body 32A, and acts as a distributed load over all of the pixels 50 disposed in the first solid block body 32A. Therefore, the pixels 50 are not adversely affected (e.g., unduly strained) by external forces. Consequently, a process for correcting the radiation image information generated by the first radiation detecting apparatus 18A, as a result of the pixels 50 being unduly strained, does not need to be carried out.

According to the present embodiment, the first solid block bodies 32A are arrayed along the irradiated surface 30a, and the heat insulator 82 is disposed in each of the first solid block bodies 32A. Consequently, temperature changes or differences developed when the radiation image information is captured, e.g., temperature differences between first solid block bodies 32A that are kept in contact with the subject 14 and other first solid block bodies 32A that are not in contact with the subject 14, do not adversely affect the pixels 50. Thus, a process for correcting the radiation image information generated by the first radiation detecting apparatus 18A, as a result of such temperature changes or differences, does not need to be carried out.

Since correcting processes are not required for compensating for strains in the pixels 50 and temperature changes or differences, the image processing operation of the console 22 can be made faster, and a simplified image processing sequence for determining whether an image recapturing process is needed or not can also be made faster. As a consequence, the image capturing process overall is made more efficient.

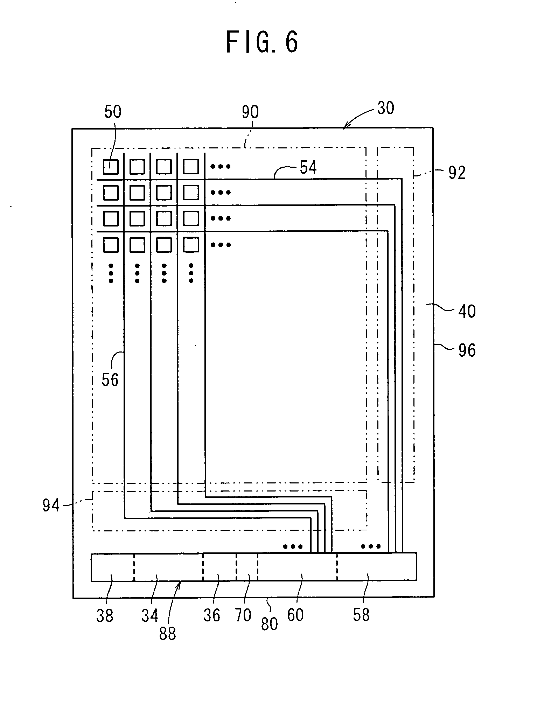

Installation of electronic circuits 88 in the first radiation detecting apparatus 18A will be described below with reference to FIGS. 6 through 9.

The flexible substrate 40 of the radiation detector 30 consists of an image capturing area 90, including the pixels 50, a first flexible interconnection section 92 including a bundle of gate lines 54 connected to the pixels 50, and a second flexible interconnection section 94 including a bundle of signal lines 56 connected to the pixels 50. Electronic circuits 88 are mounted on an end portion of the flexible substrate 40.

The electronic circuits 88 include the controller 36, the transceiver 38, the row selection scanner 58, the column selection scanner 60, the A/D converter 70, and the battery 34. The first flexible interconnection section 92 is disposed in a region lying between the image capturing area 90 and a side 96 of the radiation detector 30, which extends perpendicularly to the side 80 of the radiation detector 30, and extends along the side 96 of the radiation detector 30. The second flexible interconnection section 94 is disposed in a region lying between the image capturing area 90 and the column selection scanner 60.

As shown in FIG. 2, the electronic circuits 88 are mounted in a region corresponding to the bottom surface of one 32Ae of the first solid block bodies 32A, which is located at least on one end of the radiation detector 30.

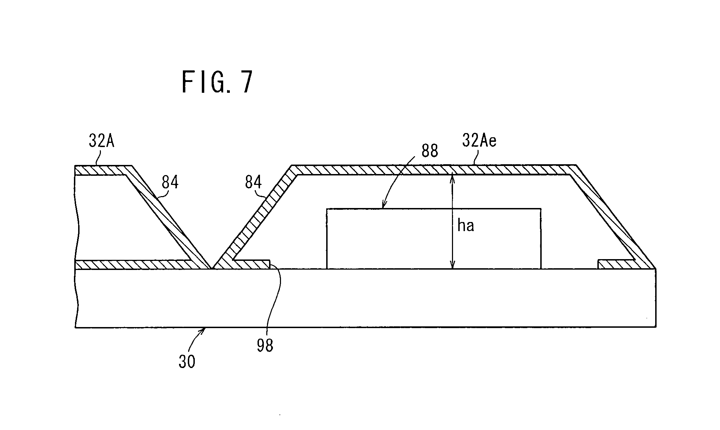

As shown in FIG. 7, the bottom surface of the first solid block body 32Ae has an opening 98 defined therein. When the first solid block body 32Ae is placed on the end of the radiation detector 30, the electronic circuits 88 enter into the first solid block body 32Ae through the opening 98 and the electronic circuits 88 are housed in the first solid block body 32Ae. The electronic circuits 88 may have a height up to an internal height ha of the first solid block body 32Ae. Therefore, the electronic circuits 88 may comprise film-like electronic circuits, or electronic circuits composed of chip-shaped electronic components, solid-state electronic devices, etc. As a result, a wider choice of circuit configurations is available for the electronic circuits 88, and hence the electronic circuits 88 can be designed with greater freedom.

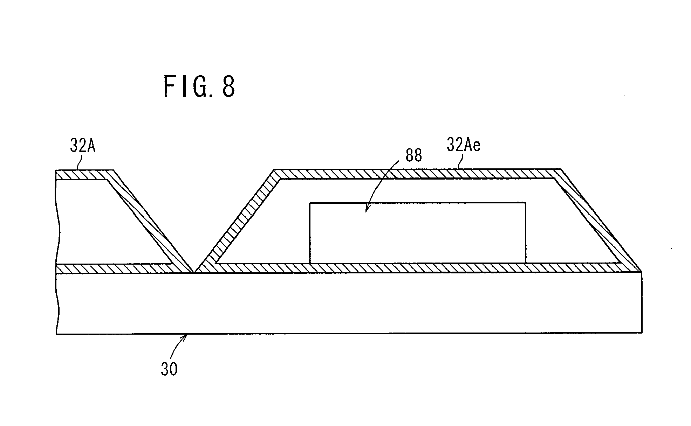

According to another example shown in FIG. 8, the electronic circuits 88 are installed in advance in the first solid block body 32Ae on the end of the radiation detector 30, and have a number of terminals (not shown) arrayed at a given pitch and extending through the bottom surface of the first solid block body 32Ae. The first and second flexible interconnection sections 92, 94 have a number of terminals (not shown) positioned on the end of the radiation detector 30, and which are arrayed at the same pitch as the respective terminals of the electronic circuits 88 that extend through the bottom surface of the first solid block body 32Ae. When the first solid block body 32Ae, with the electronic circuits 88 housed therein, is placed on the end of the radiation detector 30, the terminals of the electronic circuits 88 and corresponding terminals of the first and second flexible interconnection sections 92, 94 are electrically connected to each other, so that the electronic circuits 88 can read radiation image information from the radiation detector 30.

With the example shown in FIG. 8, since the electronic circuits 88 do not need to be separately installed on the radiation detector 30, the radiation detector 30 can be manufactured easily. If the terminals of the electronic circuits 88 are set in connectors having preset terminal pitches, the terminals of the electronic circuits 88 and corresponding terminals of the first and second flexible interconnection sections 92, 94 can be electrically connected to each other easily.

A lead plate, not shown, attached to an outer or inner side of the top surface of the first solid block body 32Ae, is effective in preventing irradiation-caused damage to the electronic circuits 88.

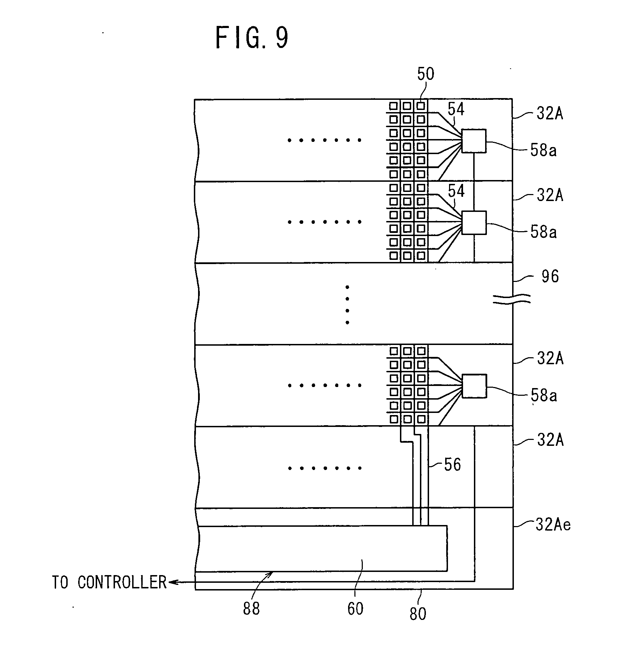

In FIGS. 6 through 8, the electronic circuits 88 are installed entirely on the end of the radiation detector 88. However, as shown in FIG. 9, the gate lines 54 may be grouped and connected to a group of row selection scanners. Specifically, the row selection scanner 58 is divided into a plurality of row selection scanners (hereinafter referred to as divided row selection scanners 58a), and the divided row selection scanners 58a are connected in series with each other. The divided row selection scanners 58a are disposed on respective ends of the first solid block bodies 32A, i.e., the divided row selection scanners 58a are disposed in a region lying between the image capturing area 90 and the side 96 of the radiation detector 30, and are spaced along the side 96 of the radiation detector 30. The divided row selection scanners 58a may be installed on the radiation detector 30, or may be housed within the respective first solid block bodies 32A. The divided row selection scanners 58a may be installed in combination with all of the first solid block bodies 32A, or may be installed in combination with only some of the first solid block bodies 32A, for example, with every other first solid block body 32A.

The gate lines 54 include gate lines assigned to each of the first solid block bodies 32A and connected to a corresponding one of the divided row selection scanners 58a. In other words, the gate lines 54 are divided into a plurality of groups, each of which is connected to one of the divided row selection scanners 58a (grouped interconnections). Lead plates, not shown, which are attached to the first solid block bodies 32A at positions corresponding to the divided row selection scanners 58a, are effective in preventing irradiation-caused damage to the divided row selection scanners 58a.

In the example shown in FIG. 9, the divided row selection scanners 58a are assigned respectively to the first solid block bodies 32A. Therefore, the number of electronic circuits 88 mounted on the end of the radiation detector 30 can be reduced, i.e., the number of pixels of the radiation detector 30 can be increased. The radiation detector 30 can thus be designed in a longer structure, for thereby capturing radiation image information of the entire spine or an entire leg of the subject 25.

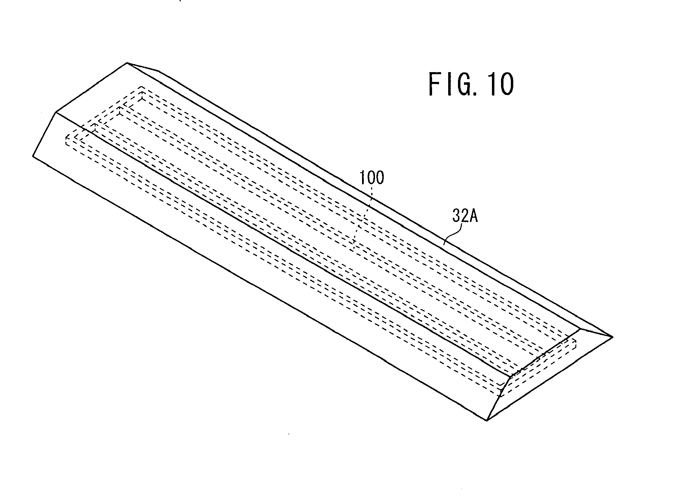

The radiation detector 30 should preferably comprise a grid for removing scattered rays of the radiation 12. According to the present embodiment, as shown in FIG. 10, a plurality of divided grids 100 are associated with the respective first solid block bodies 32A over the image capturing area 90 (see FIG. 6), and each of the divided grids 100 is disposed in one of the first solid block bodies 32A. The divided grids 100 are positioned in the image capturing area 90, exclusive of the electronic circuits 88. The size (area) of each of the divided grids 100 is substantially equal to the area of the image capturing area 90 divided by the number of first solid block bodies 32A, which are associated with the divided grids 100. The flexible first radiation detecting apparatus 18A thus incorporates the grids 100 embedded therein.

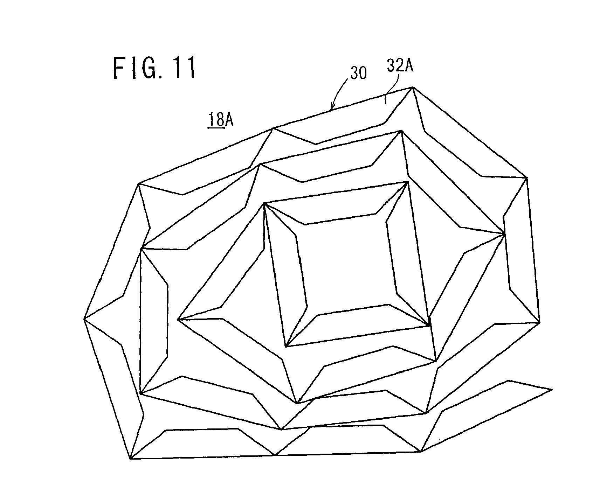

If the first radiation detecting apparatus 18A is not in use, the first radiation detecting apparatus 18A may be wound spirally into a compact tubular shape, as shown in FIG. 11, for storage in either an upstanding attitude or horizontally on a shelf. Therefore, the radiation image capturing system 10 provides an inspection space in which the first radiation detecting apparatus 18A can be compactly stored, and allows the operator to work smoothly when capturing radiation images.

A flexible handle may be joined to the longitudinal edge of the first solid block body 32Ae on the end of the radiation detector 30, and a hook may be attached to the reverse side of the radiation detector 30. When the first radiation detecting apparatus 18A is wound spirally, the handle engages with the hook in order to keep the first radiation detecting apparatus 18A spirally wound. Accordingly, the first radiation detecting apparatus 18A can conveniently be placed in storage.

The first radiation detecting apparatus 18A and the radiation image capturing system 10 according to the present invention basically are constructed as described above. Operations of the first radiation detecting apparatus 18A and the radiation image capturing system 10 will be described below.

Patient information of the patient (subject) 14 to be imaged is registered in the console 22 prior to commencement of the image capturing process. If a region to be imaged and an image capturing method are already known, then such image capturing conditions also are registered in the console 22.

For capturing radiation image information of the patient 14 while the doctor performs a surgical operation on the patient 14 in an operating room, when the doctor examines the patient 14, or when the doctor goes on rounds in the hospital, the doctor or a radiological technician places the first radiation detecting apparatus 18A between the patient 14 and the bed, with the first solid block bodies 32A positioned to face the radiation source 16.

If the chest or spine of the patient 14 is to be imaged, then the first radiation detecting apparatus 18A is placed such that the longitudinal direction of the first solid block bodies 32A runs alongside the longitudinal axis of the patient 14. Thus, the first radiation detecting apparatus 18A is deformed in its entirety into an arcuate shape, which matches substantially with the surface shape of the back of the patient 14.

Then, after having moved the radiation source 16 to a position facing the first radiation detecting apparatus 18A, the doctor or a radiological technician operates an image capturing switch of the radiation source 16 in order to start the image capturing process. When the image capturing switch is operated, the radiation source 16 sends a request to the console 22 to transmit image capturing conditions by way of wireless communications. In response to this request, the console 22 transmits the image capturing conditions to the radiation source 16 concerning the region to be imaged of the patient 14. When the radiation source 16 receives the image capturing conditions, the radiation source 16 applies radiation 12 to the patient 14 at a dosage according to the image capturing conditions.

The radiation 12 having passed through the patient 14 is applied to the grids 100 of the first radiation detecting apparatus 18A, which remove scattered rays from the radiation 12. Then, the radiation 12 is applied to the radiation detector 30. The scintillator 42 of the radiation detector 30 emits visible light at an intensity, which depends on the intensity of the applied radiation 12. The pixels 50 of the photoelectric transducer layer 46 convert the visible light into electric signals and store the electric signals as electric charges. The stored electric charges, which represent radiation image information of the patient 14, are read from the pixels 50 according to address signals, which are supplied from the address signal generator 72 of the controller 36 to the row selection scanner 58 and to the multiplexer 66 of the column selection scanner 60.

Specifically, in response to the address signal supplied from the address signal generator 72, the address decoder 67 of the line selection scanner 58 outputs a selection signal to select one of the switches SW1, which supplies a control signal Von to the gates of the TFTs 52 that are connected to the gate line 54 corresponding to the selected switch SW1. In response to the address signal supplied from the address signal generator 72, the address decoder 68 of the multiplexer 66 outputs a selection signal to successively turn on the switches SW2, so as to switch between the signal lines 56 for thereby reading, through the signal lines 56, the electric charges stored in the pixels 50 connected to the selected gate line 54.

The electric charges read from the pixels 50 connected to the selected gate line 54 are amplified by respective amplifiers 62, sampled by the sample and hold circuits 64, and supplied to the multiplexer 66. Based on the supplied electric charges, the multiplexer 66 generates and supplies a radiation image signal to the A/D converter 70, which converts the radiation image signal into a digital signal. The digital signal, which represents the radiation image information, is stored in the image memory 74 of the controller 36.

Similarly, the address decoder 67 of the line selection scanner 58 successively turns on the switches SW1 to switch between the gate lines 54 according to the address signal supplied from the address signal generator 72. The electric charges stored in the pixels 50, which are connected to the successively selected gate lines 54, are read through the signal lines 56, and processed by the multiplexer 66 and the A/D converter 70 into digital signals, which are then stored in the image memory 74 of the controller 36.

The radiation image information stored in the image memory 74 is transmitted from the transceiver 38 to the console 22 by way of wireless communications. The console 22 performs predetermined image processing on the received radiation image information, and stores the processed radiation image information in a memory, in association with registered patient information of the patient 14. The processed radiation image information is transmitted from the console 22 to the display device 20, which displays a radiation image based on the radiation image information.

As described above, the first radiation detecting apparatus 18A can prevent excessive deformations from occurring in the radiation detector 30, and prevents image distortions of the radiation image information generated by the radiation detector 30. The first radiation detecting apparatus 18A also prevents disconnections in the radiation detector 30, is of increased reliability during operation thereof, and can be used and stored highly conveniently.

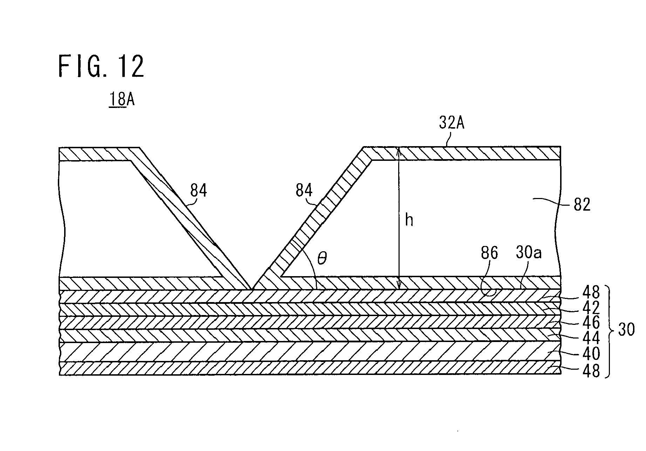

In the first radiation detecting apparatus 18A, the scintillator 42, the TFT layer 44, and the photoelectric transducer layer 46 are successively stacked in this order on the flexible substrate 40. Alternatively, the photoelectric transducer layer 46, the TFT layer 44, and the scintillator 42 are successively stacked in this order on the irradiated surface 30a. The photoelectric transducer layer 46 can efficiently convert visible light generated by the scintillator 42 into electric signals. As a result, the first radiation detecting apparatus 18A can produce high-quality radiation image information. The stacked layer structure shown in FIG. 3 may be replaced with a different stacked layer structure, as shown in FIG. 12. In the stacked layer structure shown in FIG. 12, the TFT layer 44, the photoelectric transducer layer 46, and the scintillator 42 are successively stacked in this order from the flexible substrate 40 toward the irradiated surface 30a.

In the first radiation detecting apparatus 18A, the first solid block bodies 32A are disposed on the irradiated surface 30a of the radiation detector 30. Alternatively, the first solid block bodies 32A may be disposed on another surface of the radiation detector 30, which is opposite to the irradiated surface 30a.

According to the present embodiment, furthermore, since signals are sent and received between the console 22, the first radiation detecting apparatus 18A, the radiation source 16, and the display device 20 by way of wireless communications, cables for transmitting and receiving signals are not required, and hence there are no cable-induced obstacles to operations performed by the doctor or the radiological technician. Therefore, the doctor and radiological technician are allowed to perform work smoothly and efficiently.

According to the present embodiment, moreover, radiation image information is captured when the doctor or a radiological technician turns on the image capturing switch of the radiation source 16. However, the radiation image information may also be captured when the doctor or radiological technician operates the console 22.

The dosage of applied radiation 12 may be converted directly into an electric signal by a photoelectric transducer layer, which comprises solid-state detectors made of a material such as amorphous selenium (a-Se). In accordance with such a modification, the light shield film 48 may be dispensed with.

Radiation image information may be acquired by a radiation detector of a light conversion type, which has a matrix of solid-state detectors incorporated therein. When radiation is applied to the solid-state detectors, an electrostatic latent image, which depends on the dosage of radiation, is stored in the solid-state detectors. For reading the stored electrostatic latent image, the radiation detector is irradiated with reading light by means of a flexible organic EL (electroluminescence) device, and the value of an electric current generated by the radiation detector is acquired as radiation image information, which is representative of the stored electrostatic latent image. Further, such a radiation detector can be reused when the radiation image information representing a residual electrostatic latent image is erased from the radiation detector by erasing light applied to the radiation detector (see Japanese Laid-Open Patent Publication No. 2000-105297).

When the first radiation detecting apparatus 18A is used in an operating room or the like, blood stains and other contaminants may be applied to the first radiation detecting apparatus 18A. Therefore, the first radiation detecting apparatus 18A may have a water-resistant, sealed structure, so that the first radiation detecting apparatus 18A can be sterilized and cleaned to remove such blood stains and contaminants and used repeatedly. A surface fastener may be interposed between the bottom surfaces of the first solid block bodies 32A and the irradiated surface 30a of the radiation detector 30, in order to make the first solid block bodies 32A separable from the radiation detector 30. When the first solid block bodies 32A and the radiation detector 30 are separated from each other, they can more easily be cleaned and sterilized. If such a surface fastener is incorporated into a light conversion type of radiation detector, then the stored radiation image information can easily be read from the radiation detector, since the first solid block bodies 32A can be removed therefrom.

The first radiation detecting apparatus 18A and an external device may communicate with each other by way of optical wireless communications using infrared rays or the like, rather than typical wireless communications using radio waves.

The radiation detector 30 described above incorporates TFTs 52 therein. The TFTs 52 may also be combined with another image capturing device, such as a CMOS (Complementary Metal-Oxide Semiconductor) image sensor or the like. Alternatively, the radiation detector 30 may be replaced with a CCD (Charge-Coupled Device) image sensor, for shifting and transferring electric charges with shift pulses corresponding to gate signals in the TFTs.

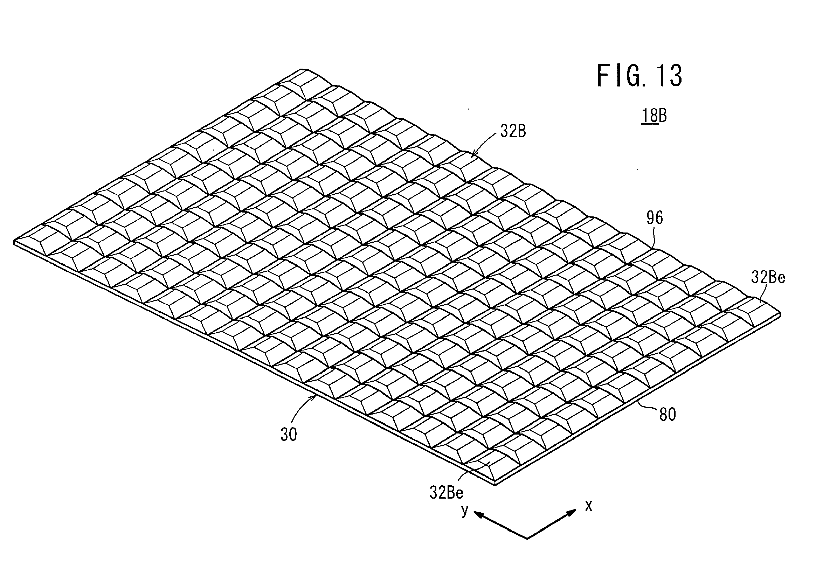

A radiation detecting apparatus according to a second specific example (hereinafter referred to as a second radiation detecting apparatus 18B) will be described below with reference to FIG. 13.

As shown in FIG. 13, the second radiation detecting apparatus 18B has essentially the same structure as the first radiation detecting apparatus 18A, but differs therefrom in that the second radiation detecting apparatus 18B includes a plurality of solid block bodies 32 made up of a matrix of second solid block bodies 32B disposed over the radiation detector 30, each of which has a square shape as seen in plan. For example, each of the second solid block bodies 32B is in the form of a quadrangular pyramidal trapezoid.

When an external force is applied to the second radiation detecting apparatus 18B, the second radiation detecting apparatus 18B is bent at boundaries between adjacent ones of the second solid block bodies 32B. More particularly, when the second radiation detecting apparatus 18B is bent in a direction to bring adjacent ones of the second solid block bodies 32B toward each other, the second radiation detecting apparatus 18B stops being bent when the mutually facing tapered surfaces 84 of the adjacent second solid block bodies 32B come into contact with each other. Since the second radiation detecting apparatus 18B is prevented from being further deformed after the adjacent tapered surfaces 84 of the second solid block bodies 32B come into contact with each other, the second radiation detecting apparatus 18B is prevented from being excessively deformed, thereby preventing disconnections from occurring in the gate lines 54 and the signal lines 56.

Unlike the first radiation detecting apparatus 18A, the second radiation detecting apparatus 18B is bendable not only in the direction indicated by the arrow y in FIGS. 2 and 13, but also in the direction indicated by the arrow x. The second radiation detecting apparatus 18B can be deformed substantially in conformity with a free-form surface, and therefore can capture a radiation image of the head, a bent elbow, a bent knee, or the like, of the patient 14.

In the second radiation detecting apparatus 18B, as with the first radiation detecting apparatus 18A, the electronic circuits 88 may be mounted in a region corresponding to the bottom surface of an array of second solid block bodies 32Be, along a side of the radiation detector 30 at an end of the radiation detector 30, or the electronic circuits 88 may be installed inside the second solid block bodies 32Be. In this case, it is desirable that the electronic circuits 88 be divided into as many circuits as the number of second solid block bodies 32Be.

The series-connected divided row selection scanners 58a may be disposed on respective ends of the second solid block bodies 32B, i.e., disposed in a region near the side 96, which is perpendicular to the side 80 of the radiation detector 30, and spaced along the side 96 of the radiation detector 30. The divided row selection scanners 58a may be installed on the radiation detector 30, or may be housed in the respective second solid block bodies 32B.

A plurality of divided grids 100 may be associated with the respective second solid block bodies 32B over the image capturing area 90 (see FIG. 6), each of the divided grids 100 being disposed in one of the second solid block bodies 32B.

As with the first radiation detecting apparatus 18A, the second radiation detecting apparatus 18B is capable of preventing excessive deformations from occurring in the radiation detector 30, and avoids image distortions in the radiation image information generated by the radiation detector 30. The second radiation detecting apparatus 18B also prevents disconnections in the radiation detector 30, is of increased reliability during operation thereof, and can be used and stored highly conveniently.

At least one first solid block body 32A and a plurality of second solid block bodies 32B may be arrayed along the radiation detector 30. For example, in the second radiation detecting apparatus 18B shown in FIG. 13, the second solid block bodies 32Be in which the electronic circuits 88 are installed may be replaced with a single first solid block body 32Ae.

## Claims

1. A radiation detecting apparatus comprising:
a flexible radiation detector (30) for detecting radiation (12) that has passed through a subject (14) and converting the detected radiation (12) into radiation image information, the flexible radiation detector (30) including a flexible substrate (40); and
a plurality of solid block bodies (32) mounted in a two-dimensional array on the flexible radiation detector (30).

2. A radiation detecting apparatus according to claim 1, further comprising:
a light shield film (48) covering at least the flexible radiation detector (30).

3. A radiation detecting apparatus according to claim 1 or 2, further comprising:
a plurality of divided grids (100) for removing scattered rays from the radiation, the divided grids (100) being associated with the solid block bodies (32), respectively,
wherein the divided grids (100) are disposed respectively in the solid block bodies (32).

4. A radiation detecting apparatus according to any one of claims 1 to 3, further comprising:
a heat insulator (82) disposed in each of the solid block bodies (32).

5. A radiation detecting apparatus according to any one of claims 1 to 4, wherein the solid block bodies (32) are disposed on a surface of the radiation detector (30) that is irradiated with the radiation (12).

6. A radiation detecting apparatus according to any one of claims 1 to 5, wherein each of the solid block bodies (32) has a tapered surface (84) facing a tapered surface (84) of an adjacent one of the solid block bodies (32), the tapered surfaces (84) of the adjacent solid block bodies (32) being spaced progressively wider from each other in a direction away from the radiation detector (30).

7. A radiation detecting apparatus according to any one of claims 1 to 6, wherein the radiation detector (30) comprises a plurality of pixels (50) disposed in areas corresponding to respective bottom surfaces (86) of the solid block bodies (32).

8. A radiation detecting apparatus according to claim 7, wherein the pixels (50) are arrayed in the areas corresponding to respective bottom surfaces (86) of the solid block bodies at a pitch (Pa), and wherein closest ones of the pixels (50) in adjacent ones of the areas are arrayed at a pitch (Pb) substantially the same as the pitch (Pa).

9. A radiation detecting apparatus according to any one of claims 1 to 8, wherein each of the solid block bodies (32) has an elongate rectangular shape as viewed in plan, which has a longer side (78) with a length equal to the length of a side (80) of the radiation detector (30), and the solid block bodies (32) are arrayed in a direction perpendicular to the side (80) of the radiation detector (30).

10. A radiation detecting apparatus according to claim 9, further comprising:
electronic circuits (88) for reading the radiation image information from the radiation detector (30), the electronic circuits (88) being disposed in one of the solid block bodies (32), which is disposed on at least one of an end of the radiation detector (30) and a region of the radiation detector (30) that corresponds to a bottom surface of one of the solid block bodies (32).

11. A radiation detecting apparatus according to claim 9, further comprising:
electronic circuits (88) for reading the radiation image information from the radiation detector (30);
the electronic circuits (88) including a plurality of pixel selecting circuits (58a) for selecting respective rows of pixels;
the pixel selecting circuits (58a) being disposed at a position near another side of the radiation detector (30), which extends perpendicularly to the side (80) of the radiation detector (30), in all or selected ones of the solid block bodies (32).

12. A radiation detecting apparatus according to claim 9, further comprising:
electronic circuits (88) for reading the radiation image information from the radiation detector (30);
the electronic circuits (88) including a plurality of pixel selecting circuits (58a) for selecting respective rows of pixels;
the pixel selecting circuits (58a) being disposed in areas of the flexible substrate (40) corresponding to bottom surfaces of all or selected ones of the solid block bodies (32) at a position near another side of the radiation detector (30), which extends perpendicularly to the side (80) of the radiation detector (30).

13. A radiation detecting apparatus according to any one of claims 1 to 8, wherein each of the solid block bodies (32) has a square shape as viewed in plan; and
the solid block bodies (32) are arrayed in a matrix over the radiation detector (30).

14. A radiation image capturing system comprising:
a radiation detecting apparatus (18) according to any one of claims 1 to 13;
a radiation source (16) for outputting the radiation (12); and
a control apparatus (22) for controlling the radiation source (16) and the radiation detecting apparatus (18).

15. A radiation image capturing system according to claim 14, wherein the radiation detecting apparatus (18) transmits the radiation image information converted by the radiation detector (30) to the control apparatus (22) by way of wireless communications.
